# EUROPEAN PATENT SPECIFICATION

(11) **EP 1 077 069 B1**
(45) Date of publication and mention of the grant of the patent: **02.05.2007**
(21) Application number: 99919561.3
(22) Date of filing: 13.05.1999
(51) Int. Cl.: A61P 1/16, A61K 45/00, A61K 39/395, A61K 38/02, A61K 31/70

(54) **PREVENTIVES/REMEDIES FOR HEPATIC CIRRHOSIS**
PRÄVENTION UND THERAPIE VON LEBERZIRRHOSEN
AGENTS PREVENTIFS/REMEDES CONTRE LA CIRRHOSE DU FOIE

(30) Priority: 14.05.1998 JP 13177198
(43) Date of publication of application: 21.02.2001
(73) Proprietor: MOCHIDA PHARMACEUTICAL CO., LTD., Shinjuku-ku Tokyo 160-8515 (JP)
(72) Inventor: UENO, Yoshiyuki, Sendai-shi, Miyagi-ken 980-0812 (JP)
(74) Representative: Grünecker, Kinkeldey, Stockmair & Schwanhäusser Anwaltssozietät
(86) International application number: PCT/JP1999/002477
(87) International publication number: WO 1999/058150

(56) References cited:
- WO-A1-95/13293
- WO-A1-97/02290
- WO-A1-97/42319
- JP-A- 9 072 901
- JP-A- 9 124 509
- HARADA K. et al., "Enhanced Apoptosis Relates to Bile Duct Loss in Primary Biliary Cirrhosis", HEPATOLOGY, 1997, Vol. 26, No. 6, pages 1399-1405, XP002922067.
- SEISHIMA M. et al., "Increased Serum Soluble Fas (sFas) Concentrations in HCV-Positive Patients with Liver Cirrhosis", JOURNAL OF HEPATOLOGY, 1997, Vol. 27, pages 424-427, XP002922068.
- GALLE P.R. et al., "Involvement of the CD95 (APO-1/Fas) Receptor and Ligand in Liver Damage", J. EXP. MED., 1995, Vol. 182, pages 1223-1230, XP002922069.
- ROUQUET N. et al., "ICE Inhibitor YVADcmk is a Potent Therapeutic Agent Against In Vivo Liver Apoptosis", CURRENT BIOLOGY, 1996, Vol. 6, No. 9, pages 1192-1195, XP002922070.

## Description

### TECHNICAL FIELD

This invention relates to the use of preventives and remedies for hepatic cirrhosis or bile duct disappearance syndrome which contain a Fas antagonist as their effective component.

### BACKGROUND ART

Fas is a cell surface protein which transmits apoptosis signal to the cell, and Fas is recognized by Fas antibody (Yonehara, S. et al., J. Exp. Med., vol. 169, 1747-1756, 1989) which is a monoclonal antibody produced by immunizing a mouse with human fibroblast. Fas gene was cloned by Itoh, N. et al., and it was then found out that Fas is a cell membrane protein of about 45 kD, and from the amino acid sequence, it was revealed that Fas is a member of TNF receptor family (Cell, vol. 66, pages 233 - 243, 1991). Mouse Fas gene was also cloned , and the expression of Fas mRNA in thymus, liver, lung, heart, and ovary was confirmed (Watanabe-Fukunaga, R. et al., J. Immunol., vol. 148, pages 1274 - 1279, 1992).

Human Fas ligand is a polypeptide which has been reported by Nagata et al. to be a native molecule which induces apoptosis of Fas-expressing cells (Takahashi, T. et al., International Immunology, vol. 6, pages 1567 - 1574, 1994). Human Fas ligand is a glycosilated type II membrane protein of TNF family with a molecular weight of about 40 kD. As in the case of TNF, human Fas ligand in the human body is estimated to be in the form of a trimer (Tanaka, M. et al., EMBO Journal, vol. 14, pages 1129 - 1135, 1995). The extracellular domain of the human Fas ligand is highly homologous with the extracellular domain of rat Fas ligand (Suda, T. et al., Cell, vol. 75, pages 1169 - 1178, 1993) and mouse Fas ligand (Takahashi, T. et al., Cell, vol. 76, pages 969 - 976, 1994). The human Fas ligand recognizes not only the human Fas but also the mouse Fas to induce the apoptosis, and vice versa, the rat Fas ligand and the mouse Fas ligand also recognize the human Fas to induce the apoptosis.

Considerable researches have also been done on the mechanism of signal transduction in the cell upon the Fas-mediated apoptosis, and identification and cloning of the factor which interacts with the intracellular domain of the Fas, in particular, the region called "death domain" to transmit or block the signal have been reported. Possibility of the involvement of interleukin-1-converting enzyme (ICE) -related thiol proteases in the signal transduction of the Fas-mediated apoptosis has also been indicated.

Relationship of the apoptosis, in particular, the Fas-mediated apoptosis with various diseases and physiological phenomena has been recently indicated. For example, possibility has been indicated for involvement of abnormal Fas-mediated apoptosis in the death of hepatocytes in viral fulminant hepatitis, in some types of autoimmune diseases, and the like.

Possibility of the involvement of the Fas/Fas ligand system in functions other than the apoptosis, for example, in the function of inducing inflammation by acting on neutrophil has also been indicated (Kayagaki, N. et al., Rinshou Meneki (Clinical Immunology), vol. 28, pages 667 - 675, 1996).

Cirrhosis is the terminal stage of a chronic progressive hepatic diseases of the liver associated with the lesion characterized by disorganization of normal hepatic lobule structure with diffuse regenerative nodules and fibrotic tissues, and cirrhosis is different from hepatitis. In the case of cirrhosis, fibrosis and nodal generation results in compression of portal vein and sinusoidal stricture, and hence, in reduced blood flow in the liver. As a result of such interference in the blood flow and insufficient supply of oxygen and substrates to the hepatocytes, failure in the function of the hepatocytes and necrosis of hepatocytes are developed, and such vicious circle is continued. The resulting morphological change in liver invites portal hypertension, esophageal varix, ascites, and other complications. Cirrhosis is etiologically categorized into viral, drug-induced, alcoholic, parasitic, congestive, biliary, and others such as Wilson's disease. (Kamata, T., Journal of Japanese Society of Internal Medicine, vol. 80, pages 1561 - 1562, 1991; Harada, T., Journal of Japanese Society of Internal Medicine, vol. 80, pages 1563 - 1567, 1991; Okudaira, M., Journal of Japanese Society of Internal Medicine, vol. 80, pages 1568 - 1571, 1991).

Etiological treatments of cirrhosis are often associated with difficulty and the main treatments have been rest and dietetic treatments for promoting the hepatocyte metabolism and regeneration to thereby reduce the speed of the hepatic disorder development. Drugs which promote protein synthesis and drugs which suppresses the fibrosis in liver have been investigated. Such investigations, however, have so far proved unsuccessful (Niwa, H. et al. ed., "Current Therapy of Gastroenterophathy '95 to '96"; Murawaki, Y. et al., Cirrhosis, pages 302 - 307, 1997).

Bile duct disappearance syndrome is a syndrome associated with diffused disappearance of hepatic bile ducts and prolonged cholestasis. The diffused bile duct disappearance is associated with various types of secondary lesions in the duct, and most of such bile duct disappearance is irreversible. Varying degree of fibrosis and infiltration of inflammatory cells including neutrophil are found in the surrounding region, and there are also found chronic cholestasis images including swelling and clearing of the liver cells, deposition of copper granules in the liver cells, and emergence of Mallory body. Finally, the lesion develops into biliary liver cirrhosis as characterized by the regenerated nodes of patchy pattern. Regeneration of bile ducts are recognized in some cases, and in such case, proliferation of irregularly branched cholangioles having narrow lumen is observed with the improvement in cholestasis. This phenomenon is believed to be the results of metaplasia of liver cells. When the disappearance of bile duct is circumscribed to the liver, atrophy and fibrosis of the liver in the lesion is observed while cholangiole reaction and cholestasis image are normally absent (Nakanuma, Y., Kan-Tan-Sui (Liver, Gallbladder, and Pancreas), vol. 26, pages 363-370, 1993).

Biliary liver cirrhosis is the condition which occurs subsequent to the cholestasis, and the liver becomes firm to exhibit granular or nodal appearance with greenish color. Fibrosis has developed in wide area surrounding the portal vein, and the portal fibrosis of one portal vein area becomes linked to the portal fibrosis of the adjacent portal vein area to surround the liver lobules, and the fiber bundle often extends to the centrilobular area. Biliary liver cirrhosis may be divided into secondary biliary cirrhosis found in association with biliary constriction or incomplete biliary obstruction in the case of biliary diseases such as cholelithiasis and chronic cholangitis, and primary biliary cirrhosis.

Primary biliary cirrhosis is a typical autoimmune disease. This disease is a cryptogenic, intractable disease wherein prolonged cholestasis is found despite the absence of extrahepatic biliary obstruction, and wherein clinical picture of cirrhosis is found in its endstage. This disease is characterized as an autoimmune disease by the presence of anti-mitochondorial antibody (AMA), and the patients are positive for antinuclear antibody and the like in some cases. The primary biliary cirrhosis is divided by the presence and the absence of subjective symptom into asymptomatic (a-PBC) and symptomatic (s-PBC). Pathologically, the lesion of the primary disorder is believed to be interlobular bile ducts of intermediate size with the diameter of 40 to 80 µm or septal ducts with larger diameter. Chronic non-suppurative destructive cholangitis (CNSDC) is generated as the early stage of the primary biliary cirrhosis, and this tissue symptom is characteristic to this disease. In the level of bile duct, the disease is associated with papillary proliferation of the epithelial cells, and transformation and necrosis and occasional exfoliation of the epithelial cells. Lymphoid aggregates with germinal centers and granuloma are often found in the area surrounding the bile duct, and sarcoidosis-like morphology is often found in association with histiocyte-like cells and epithelioid cells. The disease is morphologically classified in accordance with Schauer classification into Stage I: the stage wherein CNSDC is found (CNSDC stage, florid duct lesion); Stage II: the stage wherein ductular proliferation becomes significant and disappearance of intralobular bile duct is observed (stage of ductular proliferation); Stage III: the stage wherein fibrosis of varying degree is observed with less inflammatory response (scarring stage) ; and Stage IV: the stage of cirrhosis (cirrhosis stage). (Inoue, K. et al., Igaku-no Ayumi (Progress in Medicine), Supplement Edition, Gastroenterophathy II, Liver, Gallbladder, and Pancreas, pages 356 - 360, 1993; Onishi, Y. et al., Gekkan Yakuji (Monthly Pharmaceutics), vol. 32, pages 151 - 157, 1990).

Current symptomatic treatment of the primary biliary cirrhosis is the use of ursodeoxycholic acid (UDCA). Continuous administration of the UDCA is required for the symptomic treatment, and full recovery is not expectable. In some cases, even the improvement in the condition is unexpectable (Konnichi-no Chiryo-Shishin (Therapeutic Guide Today) 1997, Hinohara S. et al. ed., Primary Biliary Cirrhosis, Tanaka, N., pages 408 - 409, 1997; Kajiyama, G. et al., Chiryo (Therapy), vol. 78, Extra edition (Standard Formulation Guide, Primary sclerosing cholangitis), pages 854 - 856, 1996).

There has been reported for the hepatocyte disorder animal model that Fas is involved in the proliferative transformation of hepatocytes; Fas expression is enhanced in various human liver diseases; there is high possibility that cytokines such as interferone γ is involved in such enhanced Fas expression; and enhanced Fas ligand is also found in such diseases (Ryou, K. et al., Gendai Iryo (Contemporary Medicine), vol. 29, pages 165 - 169, 1997). There is also estimated in this report that Fas system is involved in viral hepatitis, and that an apoptotic mechanism different from the hepatitis is involved in the case of alcoholic cirrhosis. With regard to the diagnosis and the therapy of the liver utilizing the Fas, there have been known an evaluation method for liver diseases wherein soluble Fas in blood collected from liver disease group is measured (JP-A 9-72901), and a therapeutic agent for hepatitis containing human Fas ligand antibody as its effective component (JP-A 9-124509). It is, however, still unknown whether the Fas/Fas ligand-mediated apoptosis is directly or indirectly involved in the pathology of the cirrhosis as described above, and no preventive and/or therapeutic agent for primary biliary cirrhosis are known that utilizes the action of the Fas antagonist.

In histological study of interlobulares and the like, Harada, K. et al. and Kuroki, T. et al. reported that observation of apoptotic cells and expression of Fas is more frequent in the liver of the primary biliary cirrhosis compared to the normal liver (Kanzo (Liver), vol. 38, suppl. page 195, 1996; Virchows Arch., vol. 429, pages 119 - 129). It is, however, still unknown whether the Fas/Fas ligand-mediated apoptosis is directly or indirectly involved in the pathology of the primary biliary cirrhosis, and no preventive and/or therapeutic agent for primary biliary cirrhosis are known that utilizes the action of the Fas antagonist.

### DISCLOSURE OF THE INVENTION

An object of the present invention is to provide a preventive and therapeutic agent for cirrhosis or bile duct disappearance syndrome which acts by the novel mechanism of Fas antagonist. More specifically, the present invention provides a preventive and therapeutic agent for cirrhosis or bile duct disappearance syndrome which contains a Fas antagonist as its effective component and a therapeutic method wherein such agent is used.

The inventors of the present invention have conducted intensive studies on the relation between the apoptosis and the cirrhosis or bile duct disappearance syndrome in order to save those suffering from such diseases, and found that the pathology is improved in the model of cirrhosis or bile duct disappearance syndrome by the apoptosis-suppressing substance such as Fas antagonist. The present invention has been completed on the bases of such finding.

Accordingly, the present invention is directed to the use of a Fas antagonist in producing a pharmaceutical for preventing and/or treating hepatic cirrhosis or bile duct disappearance syndrome.

### BEST MODE FOR CARRYING OUT THE INVENTION

The present invention is hereinafter described in further detail.

The cirrhosis which may be treated by the preventive and therapeutic agent of the present invention include various diseases. The cirrhosis may be etiologically categorized into autoimmune cirrhosis, biliary cirrhosis, viral cirrhosis, alcoholic cirrhosis, cirrhosis induced by abnormal metabolism, cirrhosis caused by congestion, cirrhosis induced by hepatic toxin, cirrhosis induced by parasites, and cirrhosis induced by malnutrition. The preventive and therapeutic agent of the present invention is preferably used in autoimmune cirrhosis and biliary cirrhosis. The bile duct disappearance syndrome which may be treated by the preventive and therapeutic agent of the present invention include various diseases. The bile duct disappearance syndrome may be etiologically categorized into those caused by genetic factors, immunological factors, infectious factors, vascular disturbance, and chemicals, and the preventive and therapeutic agent of the present invention is adapted for use in all of these diseases. Synonyms for the bile duct disappearance syndrome include "syndrome of disappearing intrahepatic bile ducts", "ductopenia", "vanishing bile duct syndrome (disease)", and "ductopenic rejection".

The autoimmune cirrhosis include primary biliary cirrhosis and lupoid hepatitis.

The biliary cirrhosis include primary biliary cirrhosis and secondary biliary cirrhosis.

The present invention is preferably used for primary biliary cirrhosis.

The viral cirrhosis include those induced by hepatitis B virus, hepatitis C virus, and other hepatitis viruses.

The cirrhosis caused by abnormal metabolism include those caused by hemochromatosis, Wilson's disease, α1-antitrypsin deficiency, porphyria, glycogen storage disease, and glycogen IV synthesis, galactosemia, congenitaltyrosinemia, hepatitis C virus, or other hepatitis viruses.

The congestive cirrhosis include cirrhosis caused by hepatic veno-occlusive disease, Budd-Chiari syndrome, and prolonged constrictive pericarditis, and congestive heart failure.

The toxic cirrhosis include cirrhosis caused by tapes poisoning, yellowed rice, aflatoxin, carbon tetrachloride, and methotrexate antibiotics.

The parasitic cirrhosis include cirrhosis caused by Japanese schistosomiasis, clonorchiasis, and liver fluke.

The cirrhosis caused by malnutrition include those caused by kwashiorkor and intestinal anastomosis.

The bile duct disappearance syndrome caused by genetic factors include those caused by intrahepatic loss of bile ducts, and loss of interlobular bile ducts.

The bile disappearance syndrome caused by immunological mechanism include those caused by primary biliary cirrhosis, primary sclerosing cholangitis, sarcoidosis, and transplantation, and in particular, those caused by liver transplantation.

The infectious bile disappearance syndrome include bacterial suppurative cholangitis caused by ascending infection of an enterobacterium such as E. coli, secondary sclerosing cholangitis, and intrahepatic loss of bile ducts caused by infection with cytomegalovirus, reovirus, EB virus, or Treponema pallidum.

The bile disappearance syndrome caused by vascular disturbance include those caused by ischemic necrosis of bile ducts by blood flow disturbance in coronary artery or other arterial vascular network, and biliary disorder.

The bile disappearance syndrome caused by chemicals include transformation, necrosis, and disappearance of bile ducts caused by sulfa drug, antibiotic, Tiopronin, Methyldopa, and the like.

In these diseases, a Fas antagonist exhibits prophylactic and therapeutic effects by suppressing the Fas-mediated apoptosis involved in each disease.

It should be noted that mammals other than human may also be treated by the agent of the present invention although the human is the most important object of the therapy.

It is conceived that the preventive and therapeutic agent of the present invention which contains the Fas antagonist as its effective component attains its object of preventing and treating the hepatic cirrhosis or the bile duct disappearance syndrome by suppressing the Fas-mediated apoptosis involved in some critical mechanism of the cirrhosis or the bile duct disappearance syndrome. It remains, however, utterly unknown at what timing and in which stage the Fas-mediated apoptosis relevant to the pathology takes place, and what are the final effects of the Fas-mediated apoptosis in the pathology. In addition, the pathology of the cirrhosis is quite different from the pathology of the hepatitis as described above. In the model described in the Example of JP-A 9-124509, supra, the hepatitis model was produced by administering mononucleus cells from the human peripheral blood and staphylococcal enteroxin B as the Fas ligand supply source to thereby compulsorily induce the apoptosis. In the cirrhosis model and the bile duct disappearance syndrome model in the Examples of the present invention, the animals were not directly administered with the apoptosis-inducing Fas substance, and therefore, if one of the factors for the pathology should have been the apoptosis, such apoptosis should have been the one which has been induced by some internal mechanism. In other words, the models of the present invention is quite different from the model of the JP-A 9-124509, and the apoptosis occurring in these models are unlikely to be induced by the same mechanism. In view of such situation, it is unlikely that the hepatitis directly caused by the hepatocyte death through apoptosis described in JP-A 9-124509 is the disease caused by the same type of apoptosis as the hepatic cirrhosis and the bile duct disappearance syndrome of the present invention. It is also unlikely that the therapeutic effects of the hepatitis directly caused by the hepatocyte death through apoptosis described in JP-A 9-124509 are realized by the same apoptosis-suppressing action as that of the therapeutic and prophylactic effects of the hepatic cirrhosis and the bile duct disappearance syndrome of the present invention.

The Fas antagonist used in the present invention is not limited to any particular type as long as it suppresses or inhibits the apoptosis.

Typical Fas antagonists are substances which are capable of suppressing the binding between the Fas and the Fas ligand. The substance employed is not limited to any particular type as long as it blocks signal generation by the Fas or transduction of the thus generated signal at some stage to thereby suppress the function or the biological action of the Fas/Fas ligand system, and in particular, the Fas-mediated apoptosis. The mechanism of such blockage may be inhibition of the action, function or expression of the Fas ligand or the Fas; interaction with the extracellular domain of the Fas ligand or the Fas; inhibition of the Fas ligand-Fas interaction; affecting the interaction between the intracellular domain of the Fas and the intracellular factor which interacts with the intracellular domain of the Fas; inhibition of the activity of the intracellular factor (for example, ICE-like protease) which is involved in the signal transduction of the Fas-mediated apoptosis, and the like. The apoptosis-suppressing substance may comprise either a high molecular weight protein compound or a low molecular weight compound.

Exemplary apoptosis-suppressing substances include substances which have the activity of suppressing the Fas-mediated apoptosis, such as a Fas derivative; an anti-Fas antibody; an anti-Fas ligand antibody; an antisense oligonucleotide for the gene of the Fas or the Fas ligand; an antisense oligonucleotide for the mRNA of the Fas or the Fas ligand; a substance which interacts with the intracellular domain of the Fas; and an ICE inhibitor.

The Fas antagonist used in the present invention is preferably the one which has the function of suppressing the Fas-mediated apoptosis such as Fas derivative, anti-Fas antibody, or anti-Fas ligand antibody.

Furthermore, the anti-Fas antibody or the anti-Fas ligand antibody is preferably the antibody whose antigen is the Fas or the Fas ligand from the animal of the species to be treated. For example, the anti-Fas antibody or the anti-Fas ligand antibody used for treating human is preferably the antibody whose antigen is the Fas or the Fas ligand from human, namely, anti-human Fas antibody or anti-human Fas ligand antibody.

The anti-Fas ligand antibody is preferably a chimeric antibody or a humanized antibody. An exemplary preferable chimeric antibody which may be used for treating a human is a chimeric antibody comprising the constant region from the human antibody and the variable region from a non-human antibody. An exemplary preferable humanized antibody which may be used for treating a human is a humanized antibody wherein the constant region and the framework region (FR) are of human origin, and the complementarity determining region (CDR) is of non-human origin.

More preferably, the anti-Fas ligand antibody used in the present invention is a reshaped human antibody wherein the CDR from the antibody of a mammal other than human such as mouse is replaced with the CDR of the human antibody. A non-human antibody is associated with biological defects when it is used in treating a human, for example, relatively short circulation half life, lack of developing various important functional properties of the immunoglobulin, and immunogenicity. Furthermore, if various mouse monoclonal antibodies or other monoclonal antibodies with the antigenicity against human are developed in future and one or more such non-human antibodies are used for once or for several times, the subsequent administration of such non-human antibody after such initial administration may be nullified due to the crossreactivity even if the subsequent therapy had no relation to the initial therapy. In such a case, the non-human antibody administered after the initial administration may even act as a hazardous substance. The chimeric antibody and the humanized antibody have obviated such defects.

The Fas antagonist used in the present invention is preferably the one which suppresses the apoptosis of the Fas-expressing cell in an appropriate assay, for example, in the assay described in International Patent Application Publication No. WO 95/13293.

The antibody used in the present invention may be either a polyclonal antibody or a monoclonal antibody, and the molecular species of the antibody used in the present invention is not particularly limited. The antibody used in the present invention may be either an antibody molecule of normal form or a fragment thereof as long as the antibody used is capable of binding to the antigen to inhibit the Fas-mediated apoptosis. Exemplary antibody fragments include Fab, F(ab')₂, Fv, and single chain Fv (scFv) which is the Fv of heavy chain linked to the Fv of light chain by an adequate linker to form a single chain. Among these, an example of the most preferable anti-Fas ligand antibody is mouse F919-9-18 antibody produced by hybridoma F919-9-18 which was originally deposited on June 22, 1995 in National Institute of Bioscience and Human Technology, Agency of Industrial Science and Technology (1-3, Higashi 1-chome, Tsukuba-shi, Ibaraki-ken, Japan) (Accession No. P-15002) and transferred from the original deposition to the international deposition on May 9, 1996 (Accession No. FERM BP-5535).

The anti-Fas ligand antibody and the anti-Fas antibody used in the present invention may be prepared by known process, for example, by the process described in International Patent Application Publication No. WO 95/13293 and International Patent Application Publication No. WO 97/02290.

The chimeric antibody which may be used in the present invention may be produced by a known chimeric antibody production process. For example, a method for producing a chimeric protein is described in Example 1 of the International Patent Application Publication No. WO 95/13293.

The humanized antibody used in the present invention may be prepared in accordance with Riechmann, L. et al. (Nature 332: 323 (1988) and European Patent Publication No. EP-A-0239400); Queen et al. (Proc. Natl. Acad. Sci. USA 86: 10029 (1989), International Patent Application Publication Nos. WO 90/07861 and WO 92/11018); Co et al. (Proc. Natl. Acad. Sci. USA 88: 2869 (1991)); Co et al. (Nature 351: 501 (1991)); Co et al. (J. Immunol. 148: 1149 (1992)), and the like.

A preferable example is humanized anti-Fas ligand antibody having the CDR derived from the murine F919-9-18 antibody, which is disclosed in the Example of International Patent Application Publication No. WO 97/02290 (Application No. PCT/JP96/01820).

The Fas derivative used in the present invention is not limited to any particular type as long as it is capable of binding at least with the Fas ligand, or capable of inhibiting the Fas ligand-mediated apoptosis. The Fas derivative may also be the one which comprises an amino acid sequence of a known Fas that has been arbitrarily mutated at one or more amino acid residues by substitution, deletion, or/and addition, and which inhibits the biological actions of the Fas/Fas ligand system, and in particular, the Fas-mediated apoptosis, with the binding activity to the Fas ligand retained. The Fas derivative may also comprise a mutant of Fas, Fas in a truncated form, a chimeric protein, a fusion protein, and a chemically modified Fas. The Fas from which the Fas derivative is derived may be the one derived from any animal species as long as above-mentioned property retained, although use of the Fas of human origin is preferred in consideration of the antigenicity.

Exemplary Fas derivatives are a known Fas from which the extracellular domain or the transmembrane domain has been deleted; a chimeric protein of the extracellular domain of a Fas and another protein such as human Fas-Fc (hFas-Fc) which is a chimeric protein of the extracellular domain of human Fas and Fc fragment of human immunoglobulin. The Fas derivative is not limited for its production process, and may be the one prepared by utilizing known Fas sequences and known gene engineering techniques. For example, the production process is described in the Example 1 of International Patent Application Publication No. WO 95/13293 and Examples of Application Publication No. WO 97/42319.

Another preferable Fas derivative is the Fas having a deletion in its N terminal. Among these, Fas derivatives, the shFas(nd29)-Fc and the shFas(nd29)-hinge (International Patent Application Publication No. WO 97/42319) coded in plasmids (pM1304 and pM1317) included in the E. coli which were originally deposited on March 14, 1996 in National Institute of Bioscience and Human Technology, Agency of Industrial Science and Technology (1-3, Higashi 1-chome, Tsukuba-shi, Ibaraki-ken, Japan) (Accession Nos. P-15514 and P-15515) and transferred from the original deposition to the international deposition on March 6, 1997 (Accession No. FERM BP-5854 and Accession No. FERM BP-5855) are derivatives including the extracellular domain of the known human Fas from which N terminal sequence of from 1st to 29th amino acid has been deleted, and these highly active derivatives are preferable examples of the effective component for the preventive and therapeutic agent of cirrhosis of the present invention.

The Fas derivatives as described above which may be used in the present invention may be confirmed for their activity to bind to the Fas ligand or their activity to suppress the Fas-mediated apoptosis by an appropriate assay method.

The antisense oligonucleotide for the gene of the Fas or the Fas ligand, or the antisense oligonucleotide for the mRNA of the Fas or the Fas ligand used in the present invention is not limited to any particular sequence as long as it inhibits the expression of the Fas or the Fas ligand, and may be, for example, the antisense oligonucleotide of the Fas ligand disclosed in Example 20 of the International Patent Application Publication No. WO 95/13293.

The preventive and therapeutic agent for hepatic cirrhosis or bile duct disappearance syndrome of the present invention can be used as a therapeutic agent for the patient suffering from the hepatic cirrhosis or bile duct disappearance syndrome. The preventive and therapeutic agent of the present invention can also be used as a prophylactic agent for those suffering from hepatic diseases.

The preventive and therapeutic agent for hepatic cirrhosis or bile duct disappearance syndrome of the present invention is characterized by its inclusion of the Fas antagonist as described above. The agent may be in the form of a pharmaceutical composition or kit wherein the Fas antagonist is appropriately combined with at least one pharmaceutical carrier or medium such as sterilized water, physiological saline, a vegetable oil, a mineral oil, a higher alcohol, a higher fatty acid, or a nontoxic organic solvent; and optional additives such as an excipient, a colorant, an emulsifier, a suspending agent, a surfactant, a solubilizer, a nonadsorptive, a stabilizer, a preservative, an antioxidative, a buffer, an isotonizing agent, or a pain relieving agent. The agent may be administered either orally, or parenterally by intravenous, intracoronary, subcutaneous, intramuscular, percutaneous, intrarectal, or topical administration or by inhalation.

Preferably, the preventive and therapeutic agent of the present invention is parenterally administered by either systemic or topical, rapid or continuous administration.

The preventive and therapeutic agent of the present invention may be administered to a human at an appropriate dose which may be determined by taking the conditions and the age of the patient as well as the administration route into consideration. For example, an adequate divided dose in the range of approximately 0.01 to 100 mg/kg may be selected in the case of systemic administration. The preventive and therapeutic agent for hepatic cirrhosis or bile duct disappearance syndrome of the present invention, however, is not limited to the administration route and the dose as described above. Two or more Fas antagonists including Fas/Fas ligand binding-suppressing reagent, Fas derivative, and anti-Fas ligand antibody may be used in combination, and in further combination with other drugs.

The preventive and therapeutic agent for hepatic cirrhosis or bile duct disappearance syndrome of the present invention may be formulated into a pharmaceutical preparation in a normal process. For example, an injection may be prepared by dissolving the purified Fas antagonist such as the Fas/Fas ligand binding-suppressing reagent or the anti-Fas ligand antibody in a medium such as physiological saline or a buffer and optionally supplementing the solution with an additive such as an anti-adsorptive. The preparation may also be in the form of a lyophilizate which is to be reconstituted before its use, and may contain any of the excipients that are generally used for facilitating the lyophilization.

The Fas antagonist used in the preventive and therapeutic agent for hepatic cirrhosis or bile duct disappearance syndrome of the present invention exhibits effects of suppressing organ and tissue disorders in hepatic cirrhosis or bile duct disappearance syndrome models, in particular, in the model of primary biliary cirrhosis as shown in the Examples. It should be noted that, in the Examples, an anti-mouse Fas ligand antibody is used in the demonstration of the therapeutic and prophylactic effects since the models used in the experiments are mouse models. Equivalent inhibitory effects may be expected for the anti-human Fas ligand antibody when used in human.

The bile duct disappearance has been reported not only for the biliary diseases such as the primary biliary cirrhosis and the primary sclerosing cholangitis, but also for previous stage of the cirrhosis including various chronic liver diseases caused by different etiology such as genetic factors (loss of interlobular bile ducts), infection (cytomegalovirus, reovirus, AIDS, and hepatitis virus), vascular disturbance, chemicals (sulfa drug, antibiotics, etc.), immunological mechanism (primary biliary cirrhosis, primary sclerosing cholangitis, and liver diseases associated with transplantation), and the like (Kan-Tan-Sui (Liver, Gallbladder, and Pancreas), vol. 26(3), pages 357 - 362, 1993). Yamada et al. has described that cirrhosis is the pathology in the end stage of the chronic liver diseases as described above (Kan-Tan-Sui (Liver, Gallbladder, and Pancreas), vol. 26(2), pages 181 - 186, 1993). In view of the reports and the descriptions as described above, it should be conceived that the Fas antagonist which shows the action of suppressing the organ and tissue disorder for the mouse primary biliary cirrhosis model used in the Examples of the present invention should be effective in preventing and treating all of the cirrhosis caused by different causes.

It should be noted that the preventive and therapeutic agent for cirrhosis or bile duct disappearance syndrome of the present invention exhibits no toxicity as demonstrated in the following Examples, and therefore, it can be used safely. In view of such situation, the preventive and therapeutic agent for cirrhosis or bile duct disappearance syndrome of the present invention is expected to exhibit prophylactic, therapeutic, or ameliorating effects for the cirrhosis or bile duct disappearance syndrome.

Next, the present invention is described in further detail by referring to Examples which are given by way of examples.

The abbreviations used in the following description are those commonly used in the art.

The production process and the apoptosis-suppressing activity of the shFas (nd29) -Fc and shFas (nd29) -hinge which are the Fas derivative contained in the Fas antagonist of the present invention are disclosed in Examples 2, 3, 5, 6, and 8 of International Patent Application Publication No. WO 97/42319. The production process and the apoptosis-suppressing activity of the anti-Fas ligand antibody which is contained in the Fas antagonist of the present invention are disclosed in International Patent Application Publication No. WO 97/02290.

### Example 1: Toxicity study of shFas(nd29)-Fc

### (1) Test Method

Male, 6 week BDF1 mice (manufactured by Charles River Japan) were administered with shFas(nd29)-Fc at a dose of 10 and 30 mg/kg once in every 2 days for 12 days, namely, for 7 times in total from their tail vein to evaluate the effect of the reagent. The experiment was conducted by dividing the mice into 3 groups each consisting of 3 animals, namely, into the control group, the group administered with 10 mg/kg of the shFas(nd29)-Fc, and the group administered with 30 mg/kg of the shFas(nd29)-Fc. In order to administer an equal amount protein, namely, 30 mg/kg of protein to each group, the control group was administered with 30 mg/kg of human serum albumin; the group administered with 10 mg/kg of the shFas (nd29)-Fc was administered with 10 mg/kg of the shFas(nd29)-Fc and 20 mg/kg of the human serum albumin; and the group administered with 30 mg/kg of the shFas(nd29)-Fc was administered with 30 mg/kg of the shFas(nd29)-Fc.

The mice were weighed once in every two days from the day of the first administration. The mice were also evaluated for their blood count by collecting the blood from their eyeground vein on the 14th day from the day of the first administration. After measuring the blood count, plasma was prepared to measure GOT, GPT, and creatinine. Autopsy was also conducted after collecting the blood and major organs (lung, heart, liver, kidney, spleen, and intestine) were visually inspected for their change in appearance. The blood count was measured with an automatic cell counter, K-2000 (Sysmex). GOT, GPT, and creatinine were measured with an autoanalyzer COBASFARA (manufactured by Roche).

### (2) Results

No significant effects were recognized in the weight increase, blood count, liver (GOT and GPT), kidney (creatinine), and other major organs (findings in visual inspection) in the mice administered with shFas(nd29)-Fc at a dose of 10 and 30 mg/kg once in 2 days for 12 days, namely, for 7 times in total. Example 2: Effects of administration of shFas(nd29)-Fc in the mouse primary biliary cirrhosis model

### (1) Preparation of primary biliary cirrhosis mouse model

The primary biliary cirrhosis mouse model was produced in accordance with the method of Howell et al. (J. Immunology, vol. 143, 476-483, 1989). Female, four week old Balb/c mice (Nippon SLC) were irradiated at 600 rad which is the medium lethal dose (day 0). The irradiated mice were transplanted from their tail vein with 3 x 10⁷ spleen lymphocytes from B10D2 mouse at 4 to 6 hours after the irradiation. At day 21 after the transplantation, liver was extirpated, and fixed in 4% paraformaldehyde. Tissue section of the fixed liver was stained with hematoxilin-eosine, and observed under microscope to check for the bile duct inflammation (cellular infiltration and tissue disorder) and to conduct the pathological evaluation.

The spleen lymphocytes from B10D2 mouse which were transplanted in the irradiated Balb/c mice were prepared as described below. The spleen of B10D2 mouse (Nippon SLC) was disintegrated in Hanks' solution (Nissui Seiyaku) using forceps, and the disintegrated spleen was centrifuged to obtain the cells. The cells were suspended in 0.017M Tris-0.747% ammonium chloride solution to lyze the unnecessary erythrocytes. The resulting cells were further washed in Hanks' solution to obtain the spleen lymphocytes from B10D2 mouse.

### (2) Administration of shFas(nd29)-Fc

The shFas(nd29)-Fc-administration group of mice were administered with the shFas(nd29)-Fc at a dose of 20 µg/0.2 ml/mouse for three times, namely, immediately before the transplantation of spleen lymphocyte from B10D2 mouse (day 0), and 1 day and 7 days after the transplantation (day 1 and day 7) from their tail vein (n = 4). The control group was administered from their tail vein with physiological saline at a dose of 0.2 ml/mouse (n = 5).

### (3) Pathological evaluation

The pathology was evaluated by scoring the observation in accordance with the criteria for cholangitis of J. Vieling as shown in Table 1.

**Table 1**

| Grade | Inflammation in portal vein | Disorder in vile duct |
|---|---|---|
| 0 | less than 30% | Normal |
| 1 | 30% to less than 40% | Inflammation in the area surrounding the bile duct |
| 2 | 40 to less than 60% | Inflammation in the area surrounding the bile duct and emergence of abnormal cells |
| 3 | 60 to less than 80% | Inflammation in the bile duct |
| 4 | 80% or more | Disturbance or destruction of the bile duct structure |

### (4) Results

The results are shown in Table 2.

**Table 2: Effects of shFas(nd29)-Fc administration on cholangitis (day 21)**

| Group | Percentage of animals exhibiting inflammation in bile duct (%) | Average pathological Score |
|---|---|---|
| Control | 100 (5/5) | 1.6 |
| shFas(nd29)-Fc | 25 (1/4) | 0.25 |

Inflammation in bile duct was found in 5 animals out of 5 animals in the control group while the inflammation was found in only 1 animal out of 4 animals in the group administered with the shFas(nd29)-Fc. The average pathological score of the group administered with the shFas (nd29) -Fc was lower than that of the control group.

### Example 3: Production of anti-mouse Fas ligand antibody and its purification

### (1) Production of anti-mouse Fas ligand antibody

A plasmid containing human elongation factor (EF) promoter (Mizushima-Nagata, Nucleic Acids Research, vol. 18, page 5322, 1990), and in its downstream, the gene coding for the chimeric protein prepared by fusing the extracellular domain of mouse Fas ligand from genetically engineered mouse Fas ligand WX2 (J. Immunology, vol. 157, pages 3918 - 3924, 1996) and the intracellular domain, the transmembrane domain, and a part of the extracellular domain (from N terminal to 78th amino acid) of mouse CD40 ligand was prepared. The plasmid was transfected in WR19L cell to obtain a recombinant cell W40LFL expressing the mouse Fas ligand on its cell membrane for use as the antigen to be administered. Armenian hamsters were used for the animals to be immunized. The Armenian hamsters were subcutaneously administered with 1 x 10⁷ W40LFL cells mixed with Freund complete adjuvant, and a month later, subcutaneously administered with 2 x 10⁷ W40LFL cells suspended in PBS, and in another a month later, administered with 5 x 10⁶ W40LFL cells suspended in PBS from their foot pad. 3 days after the administration, lymph node cells were collected and fused with mouse myeloma cell P3-X63-Ag8-U1 (P3-U1). After selecting the hybridoma by HAT medium (hypoxanthine-aminopterin-thymidine), hybridoma FLIM58 whose supernatant had neutralizing activity for cytotoxicity of mouse Fas ligand was obtained from the survived hybridomas.

### (2) Production of FLIM58 and its purification

Hybridoma FLIM58 was cultivated in serum-free medium Hybridoma-SFM (GIBCO BRL), and the culture supernatant was purified by protein A column (PROSEP-A, Bioprocessing) to obtain purified antibody FLIM58. Concentration of the protein was calculated from absorbance at 280 nm.

### Example 4: Toxicity study of the anti-mouse Fas ligand antibody FLIM58

### (1) Method

Male, 8 week old DBA/1J mice and C3H/He mice (Charles River Japan) were used. The mice were administered from their tail vein with the anti-mouse Fas ligand antibody FLIM58 at a dose of 100 mg/30 ml/kg. The control group was administered from their tail vein with physiological saline at a dose of 30 ml/kg. The group consisted of three animals for both strains. Observation period was 7 days, and body weight measurement, hematological tests (red blood cell, white blood cell, platelet), and hematobiological tests (GOT, GPT, urea nitrogen), and autopsy with naked eye were conducted.

### (2) Results

The body weight increase, the hematological test values (red blood cell, white blood cell, platelet), and the hematobiological test values (GOT, GPT, urea nitrogen) of the group administered with the anti-mouse Fas ligand antibody FLIM58 were not significantly different from those of the control group. In addition, no abnormalities were found in the group administered with the anti-mouse Fas ligand antibody FLIM58 by the autopsy with naked eye.

### Example 5: Effects of administration of anti-mouse Fas ligand antibody FLIM58 in the primary biliary cirrhosis mouse model

### (1) Preparation of primary biliary cirrhosis mouse model

The primary biliary cirrhosis mouse model was produced by repeating the procedure of Example 2.

### (2) Administration of FLIM58

The FLIM58 administration group of mice were administered with the FLIM58 at a dose of 0.2 ml/mouse for three times, namely, immediately before the transplantation of spleen lymphocyte from B10D2 mouse (day 0), and 1 day and 7 days after the transplantation (day land 7) from their tail vein (n = 5). The control group was administered from their tail vein with physiological saline at a dose of 0.2 ml/mouse (n = 5).

### (3) Pathological evaluation

The pathology was evaluated as in the case of Example 2.

### (4) Results

The results are shown in Table 3.

**Table 3: Effects of FLIM58 administration on cholangitis (day 21)**

| Group | Percentage of animals exhibiting inflammation in bile duct (%) | Pathological Score |
|---|---|---|
| Control | 100 (5/5) | 1.6 |
| FLIM58 | 40 (2/5) | 0.6 |

Inflammation in bile duct was found in 5 cases out of 5 animals in the control group while the inflammation was found in only 2 cases out of 5 animals in the group administered with the FLIM58. The score of the group administered with the FLIM58 was lower than that of the control group.

### INDUSTRIAL APPLICABILITY

The preventive and therapeutic agent for cirrhosis or bile duct disappearance syndrome of the present invention contains a Fas antagonist as its effective component, and it has the action of suppressing the Fas-mediated apoptosis. Therefore, the agent of the present invention has the effects of preventing or treating the Fas-mediated cirrhosis or bile duct disappearance syndrome wherein the biological actions such as Fas-mediated cell death where Fas/Fas ligand system is involved. The Fas antagonist of the present invention is highly expected for use as a prophylactic and therapeutic agent for the cirrhosis or bile duct disappearance syndrome wherein the Fas-mediated apoptosis is involved.

## Claims

1. Use of a Fas antagonist in producing a pharmaceutical for preventing and/or treating hepatic cirrhosis or bile duct disappearance syndrome.

2. The use of claim 1, wherein said Fas antagonist is a substance which suppresses binding between Fas and Fas ligand.

3. The use of claim 1 or 2, wherein said Fas antagonist is a Fas derivative.

4. The use of claim 1 or 2, wherein said Fas antagonist is an anti-Fas ligand antibody.

5. The use of any of claims 1 to 4, wherein said hepatic cirrhosis is biliary cirrhosis.

6. The use of claim 5, wherein said hepatic cirrhosis is primary biliary hepatic cirrhosis.

7. The use of any of claims 1 to 4, wherein said bile duct disappearance syndrome is bile duct disappearance syndrome caused by an immunological mechanism.

## Patentansprüche

1. Verwendung eines Fas-Antagonisten zur Herstellung eines Medikaments zur Verhinderung und/oder Behandlung von Leberzirrhose oder Gallengangsverlustsyndrom (bile duct disappearance syndrome).

2. Die Verwendung von Anspruch 1, wobei der Fas-Antagonist eine Substanz ist, die die Bindung zwischen Fas und Fas-Ligand unterdrückt.

3. Die Verwendung von Anspruch 1 oder 2, wobei der Fas-Antagonist ein Fas-Derivat ist.

4. Die Verwendung von Anspruch 1 oder 2, wobei der Fas-Antagonist ein anti-Fas Liganden Antikörper ist.

5. Die Verwendung gemäß einem der Ansprüche 1 bis 4, wobei die Leberzirrhose eine biliäre Leberzirrhose ist.

6. Die Verwendung von Anspruch 5, wobei die Leberzirrhose eine primäre biliäre Leberzirrhose ist.

7. Die Verwendung gemäß einem der Ansprüche 1 bis 4, wobei das Gallengangsverlustsyndrom, ein Gallengangsverlustsyndrom ist, das durch einen immunologischen Mechanismus verursacht wurde.

## Revendications

1. Utilisation d'un antagoniste de Fas dans la production d'un produit pharmaceutique pour la prévention et/ou le traitement d'une cirrhose hépatique ou d'un syndrome de disparition des voies biliaires.

2. Utilisation selon la revendication 1, dans laquelle ledit antagoniste de Fas est une substance qui supprime la liaison entre Fas et le ligand de Fas.

3. Utilisation selon la revendication 1 ou 2, dans laquelle ledit antagoniste de Fas est un dérivé de Fas.

4. Utilisation selon la revendication 1 ou 2, dans laquelle ledit antagoniste de Fas est un anticorps anti-ligand de Fas.

5. Utilisation selon l'une quelconque des revendications 1 à 4, dans laquelle ladite cirrhose hépatique est une cirrhose biliaire.

6. Utilisation selon la revendication 5, dans laquelle ladite cirrhose hépatique est une cirrhose hépatique biliaire primaire.

7. Utilisation selon l'une quelconque des revendications 1 à 4, dans laquelle ledit syndrome de disparition des voies biliaires est un syndrome de disparition des voies biliaires provoqué par un mécanisme immunologique.
